# EUROPEAN PATENT SPECIFICATION

(11) **EP 3 110 244 B1**
(45) Date of publication and mention of the grant of the patent: **15.08.2018**
(21) Application number: 15711323.4
(22) Date of filing: 25.02.2015
(51) Int. Cl.: A01K 41/00, A01K 43/00

(54) **PROCESS FOR PRODUCING YOUNG CHICKEN**
VERFAHREN ZUR PRODUKTION VON JUNGEN HÜHNERN
PROCÉDÉ DE PRODUCTION DE JEUNES POULETS

(30) Priority: 25.02.2014 NL 2012322
(43) Date of publication of application: 04.01.2017
(73) Proprietor: HatchTech Group B.V., 3905 TB Veenendaal (NL)
(72) Inventor: METER, Tjitze, NL-3903 DH Veenendaal (NL)
(74) Representative: Nederlandsch Octrooibureau
(86) International application number: PCT/NL2015/050116
(87) International publication number: WO 2015/130166

(56) References cited:
- WO-A1-98/24327
- WO-A2-2007/110861
- FR-A1- 2 972 896
- US-A1- 2003 228 313
- US-A1- 2013 319 335

## Description

### Background

The present invention relates to a method for producing chicken at a hatchery, the method comprising;
- incubating a batch of eggs in an incubating device during an incubating period of between about 7 to about 20 days, and then
- for each egg of the batch, detecting the presence of a hatchable chicken embryo,
- dividing the batch of eggs into at least a hatchable batch portions and a not-hatchable batch portion,
- hatching the hatchable batch portion until hatching occurs,
- rearing of chicken hatched from said hatchable batch portion.

In this connection WO2007110861, titled APPARATUS AND METHOD FOR FEEDING CHICKS DURING THE HATCHING PROCESS, discloses a method and a compartment within an incubator, capable of allowing chicks to feed during the hatching process without reducing hatchability or liveability. Thanks to immediate post hatch nutrition, the development during the chicks first days is significantly improved, resulting in a significant improvement in their weight as well.

It is known in the art to dispose not hatchable eggs of the hatchery and process these not hatchable eggs into animal feed.

### Summary of the invention

The invention aims to provide a method for producing chicken at a hatchery wherein the method has a reduced number of in- and outgoing process flows.

Another object of the invention is to improve a method for producing chicken at a hatchery in that a problem associated with known methods is at least partly solved. Yet another object of the invention is to provide an alternative method for producing chicken at a hatchery.

According to a first aspect of the invention this is realized with a method for producing chicken at a hatchery, the method comprising;
- incubating a batch of eggs in an incubating device during an incubating period of between about 7 to about 20 days, and then,
- for each egg of the batch, detecting the presence of a hatchable chicken embryo,
- based on the presence of a hatchable chicken embryo, dividing the batch of eggs into at least a hatchable batch portions and a not-hatchable batch portion,
- hatching the hatchable batch portion in a hatching device until hatching occurs,
- rearing of chicken hatched from said hatchable batch portion,
wherein the method comprises at the hatchery processing the not-hatchable batch portion into feed and administering said feed at the hatchery to hatched chickens during rearing.

The at the hatchery processing the not-hatchable batch portion into feed and administering said feed at the hatchery to hatched chickens during rearing reduces the number of in- and outgoing process flows because at the hatchery an ingoing flow of feed as well as an outgoing flow of not-hatchable eggs is at least partly omitted.

"At the hatchery" defines a site where eggs are hatched to chicks between 0 to 4 days old. Thereafter, these 0 to 4 days old chicks are transported away from the hatchery to breeding farms where the chicks grow further. It is conceivable that incubating a batch of eggs in an incubating device during an incubating period of between about 7 to about 20 days, takes place somewhere else remote from the hatchery.

Detecting the presence of a hatchable chicken embryo, refers to a step which is known in the art as candling of eggs. The presence of an embryo is detected from the outside of the egg. The detecting step leads to an estimate whether the embryo may be successfully hatched or not. In other words the egg is hatchable or not.

In this respect, rearing refers to initial rearing shortly after hatching.

In an embodiment the method comprises administering only said feed at the hatchery, to hatched chickens during rearing, such that additional nutrients are not required at least for an initial rearing period of about 1 to 4 days. This even more omits an ingoing flow of feed. It will be clear that "only" refers to said feed. There may be other matters administered to the chicken as desired, like medicine.

In an embodiment, the method comprises at the hatchery processing the entire not-hatchable batch portion into feed and administering said feed at the hatchery to hatched chickens during rearing such that no process flow of eggs or feed leaves the hatchery. This even more omits an outgoing flow of not-hatchable eggs.

In an embodiment, the rearing of chicken hatched from said hatchable batch portion comprises rearing the newly hatched chickens in the hatching device, and the administering said feed at the hatchery to hatched chickens comprises administering said feed in the hatching device during hatching. The administering said feed in the hatching device during hatching facilitates early feeding which promotes chick quality. During hatching means that feed is already administered if hatching starts.

In an embodiment, the method comprises rearing the newly hatched chicken in the hatching device during an initial rearing period of between 0 to 4 days.

In an embodiment of the method, the at the hatchery processing the not-hatchable batch portion into feed comprises sterilizing eggs and/or egg components. This even more facilitates the processing of the not-hatchable batch portion into feed.

In an embodiment of the method, the at the hatchery processing the not-hatchable batch portion into feed comprises one or more of;
- selecting eggs,
- breaking eggs to obtain a mixture of egg shells and egg contents,
- separating egg contents into shell, yolk and albumen,
- disinfecting egg components,
- cooking eggs,
- processing eggs into a crumbly substance,
- pelletizing eggs.

In an embodiment, the at the hatchery processing the not-hatchable batch portion into feed comprises adding nutrients and/or other animal feed substances for supplementing said feed.

In an embodiment, the administering said feed at the hatchery to hatched chickens comprises administering said feed to chickens hatched from said hatchable batch portion of eggs.

In an embodiment, the administering said feed at the hatchery to hatched chickens comprises administering said feed to chickens hatched from a subsequent or preceding batch of eggs. This allows to take into account the time needed to process the not-hatchable batch portion into feed.

In an embodiment of the method, the detecting the presence of a hatchable chicken embryo comprises one or more of heartbeat detection with respect to an egg, candling of an egg, thermal imaging of an egg, colour measurement of an egg, weighing of an egg. Preferably, then the detecting is followed by, based on the presence of a not-hatchable chicken embryo and colour or weight of an egg, dividing the not-hatchable batch portion into at least a first not-hatchable batch portion and a second not-hatchable batch portion, wherein eggs of the first not-hatchable batch portion are non-rotten and contain no embryo or an embryo smaller than about 20 mm, and eggs of the second not-hatchable batch portion are rotten and/or contain an embryo larger than about 20 mm.

In an embodiment, the method comprises processing the first not-hatchable batch portion into feed.

The invention further relates to a device comprising one or more of the characterising features described in the description and/or shown in the attached drawings.

The invention further relates to a method comprising one or more of the characterising features described in the description and/or shown in the attached drawings.

The various aspects discussed in this patent can be combined in order to provide additional advantages.

### Description of the drawings

The invention will be further elucidated referring to the drawings wherein shown in:
Fig. 1 a flow diagram of a known method for producing chicken at a hatchery; and
Fig. 2 a flow diagram of a methods for producing chicken according to the invention;

### Detailed description of embodiments

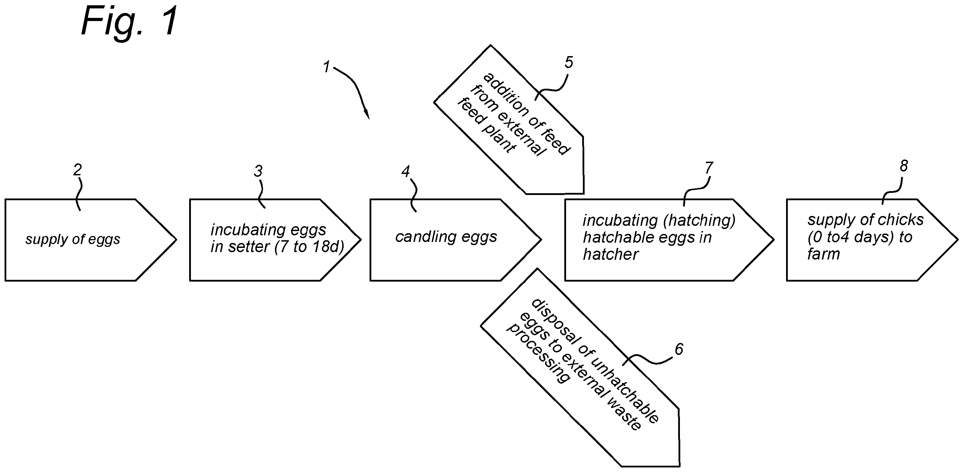

In the figure 1 a known method 1 for producing chicken at a hatchery is shown. A main flow 2,3,4,7,8, and secondary flows 5 and 6 can be distinguished. The main flow is the process at the hatchery from egg to newly hatched chicken. One secondary flows is the ingoing flow 5 of feed from an external feed plant away from the hatchery. Another secondary flow is the outgoing flow 6 of unhatchable eggs to an external waste processing away from the hatchery.

The main flow 2,3,4,7,8 will now be described. A batch of eggs is supplied 2 from a site to the hatchery. The batch of eggs is then incubated 3 in an incubating device like a setter during an incubating period of between about 7 to about 20 days.

Subsequently, for each egg of the batch of eggs, the presence of a hatchable chicken embryo is detected 4 by using candling technology which is known in the art of producing chicken. Any suitable technology will suffice, like e.g. heartbeat detection with respect to an egg, and thermal imaging of an egg.

Based on the outcome of the detection, thus on the presence of a hatchable chicken embryo, the batch of eggs is divided into a hatchable batch portions and a not-hatchable batch portion. The hatchable batch portion is then hatched 7 until hatching occurs.

Finally, the method 1 ends when the chicken that have hatched from the hatchable batch portion are transported 8 to a breeding farm away from the hatchery. The chicken are between 0 to 4 days when they leave the hatchery.

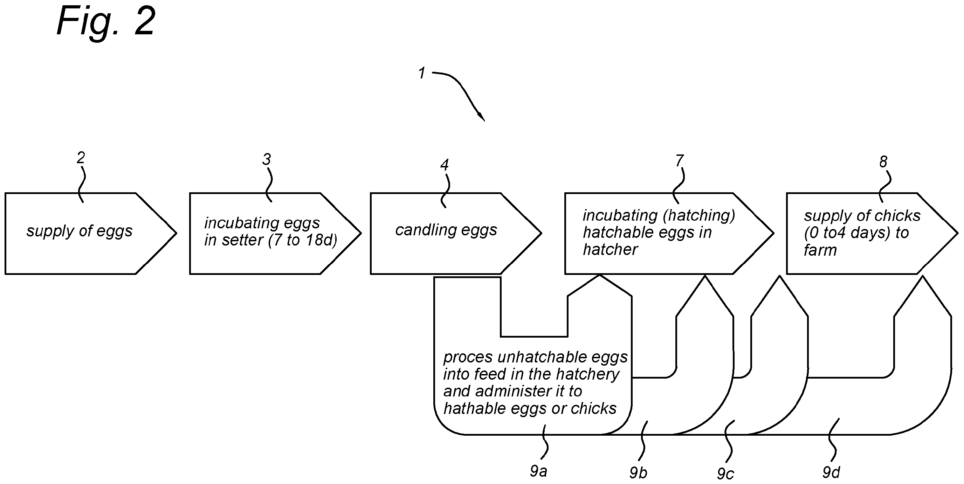

Fig. 2 shows a flow diagram of a method 1 for producing chicken according to the invention. In general only differences compared with the known method of fig. 1 are described.

As shown according to the invention an ingoing flow of feed as well as an outgoing flow of not-hatchable eggs is omitted.

Instead, the method 1 comprises at the hatchery processing 9a the not-hatchable batch portion into feed. The feed is then administering 9b, 9c, 9d at the hatchery to hatched chickens during rearing. Here, the feed is administered 9b in the hatching device during hatching which promotes taking in of feed by newly hatched chicken as soon as possible.

In this case only feed originating from the not-hatchable batch portion is administered at the hatchery such that a closed system is obtained with respect to feed. This eases the process in terms of traceability and transportation. No or less process flow of eggs or feed leaves the hatchery. In addition, costs are reduced since the not-hatchable batch portion is recycled.

Preferably said feed is administered to chickens hatched from said hatchable batch portion of eggs, it is however conceivable that said feed is administered to chickens hatched form a subsequent batch of eggs. As will be understood, the processing 9a of the not-hatchable batch portion into feed will take its time of passage parallel to the main flow 2, 3, 4, 7, 8.

It will also be obvious after the above description and drawings are included to illustrate some embodiments of the invention, and not to limit the scope of protection. Starting from this disclosure, many more embodiments will be evident to a skilled person which are within the scope of protection and the essence of this invention and which are obvious combinations of prior art techniques and the disclosure of this patent.

## Claims

1. Method (1) for producing chicken at a hatchery, the method comprising;
- incubating a batch of eggs (3) in an incubating device during an incubating period of between about 7 to about 20 days, and then,
- for each egg of the batch, detecting (4) the presence of a hatchable chicken embryo,
- based on the presence of a hatchable chicken embryo, dividing the batch of eggs into at least a hatchable batch portions and a not-hatchable batch portion,
- hatching (7) the hatchable batch portion in a hatching device until hatching occurs,
- rearing of chicken hatched from said hatchable batch portion,
**characterized in that**, the method comprises at the hatchery processing the not-hatchable batch portion into feed and administering (9a) said feed at the hatchery to hatched chickens during rearing.

2. Method according to claim 1, comprising administering only said feed at the hatchery, to hatched chickens during rearing, such that additional nutrients are not required at least for an initial rearing period of about 1 to 4 days.

3. Method according to a preceding claim, wherein the method comprises at the hatchery processing the entire not-hatchable batch portion into feed and administering said feed at the hatchery to hatched chickens during rearing such that no process flow of eggs or feed leaves the hatchery.

4. Method according to a preceding claim, wherein the rearing of chicken hatched from said hatchable batch portion comprises rearing the newly hatched chickens in the hatching device, and the administering said feed at the hatchery to hatched chickens comprises administering said feed in the hatching device during hatching.

5. Method according to claim 4, comprising rearing the newly hatched chicken in the hatching device during an initial rearing period of between 0 to 4 days.

6. Method according to a preceding claim, wherein at the hatchery processing the not-hatchable batch portion into feed comprises sterilizing eggs and/or egg components.

7. Method according to claim 6, wherein at the hatchery processing the not-hatchable batch portion into feed comprises one or more of;
- selecting eggs,
- breaking eggs to obtain a mixture of egg shells and egg contents,
- separating egg contents into shell, yolk and albumen,
- disinfecting egg components,
- cooking eggs,
- processing eggs into a crumbly substance,
- pelletizing eggs.

8. Method according to claim 7, comprising adding nutrients and/or other animal feed substances for supplementing said feed.

9. Method according to a preceding claim, wherein the administering said feed at the hatchery to hatched chickens comprises administering said feed to chickens hatched from said hatchable batch portion of eggs.

10. Method according to a preceding claim, wherein administering said feed at the hatchery to hatched chickens comprises administering said feed to chickens hatched from a subsequent or preceding batch of eggs.

11. Method according to a preceding claim, wherein the detecting the presence of a hatchable chicken embryo comprises one or more of heartbeat detection with respect to an egg, candling of an egg, thermal imaging of an egg, colour measurement of an egg, weighing of an egg.

12. Method according to claim 11, comprising, based on the presence of a not-hatchable chicken embryo and colour or weight of an egg, dividing the not-hatchable batch portion into at least a first not-hatchable batch portion and a second not-hatchable batch portion, wherein eggs of the first not-hatchable batch portion are non-rotten and contain no embryo or an embryo smaller than about 20 mm, and eggs of the second not-hatchable batch portion are rotten and/or contain an embryo larger than about 20 mm.

13. Method according to claim 12, comprising processing the first not-hatchable batch portion into feed.

## Patentansprüche

1. Verfahren (1) zum Erzeugen von Hühnern in einer Brutstätte, worin das Verfahren umfasst:
- Inkubieren einer Charge von Eiern (3) in einer Inkubationsvorrichtung während einer Inkubationszeit von etwa 7 bis etwa 20 Tagen, und dann
- Ermitteln (4) des Vorhandenseins eines bebrütbaren Hühnerembryos für jedes Ei der Charge,
- basierend auf dem Vorhandensein eines bebrütbaren Hühnerembryos, Aufteilen der Charge von Eiern in mindestens einen bebrütbaren Chargenanteil und einen nicht-bebrütbaren Chargenanteil,
- Bebrüten (7) des bebrütbaren Chargenanteils in einer Brutvorrichtung bis das Schlüpfen eintritt,
- Aufzucht der geschlüpften Hühner dieses bebrütbaren Chargenanteils,
**dadurch gekennzeichnet, dass** das Verfahren das Verarbeiten der nicht-bebrütbaren Eier zu Futter in der Brutstätte und das Verabreichen (9a) dieses Futters in der Brutstätte an die geschlüpften Hühner während der Aufzucht umfasst.

2. Verfahren nach Anspruch 1, umfassend das Verabreichen nur dieses Futters in der Brutstätte an die geschlüpften Hühner während der Aufzucht, so dass zusätzliche Nährstoffe für mindestens einen anfänglichen Aufzuchtzeitraum von etwa 1 bis 4 Tagen nicht erforderlich sind.

3. Verfahren nach einem vorhergehenden Anspruch, worin das Verfahren das Verarbeiten des gesamten nicht-bebrütbaren Chargenanteils zu Futter in der Brutstätte und das Verabreichen dieses Futters an die geschlüpften Hühner in der Brutstätte während der Aufzucht umfasst, so dass kein Verfahrensablauf von Eiern oder Futter die Brutstätte verlässt.

4. Verfahren nach einem vorhergehenden Anspruch, worin die Aufzucht von Hühnern, die aus dem bebrütbaren Chargenanteil geschlüpft sind, die Aufzucht der neu-geschlüpften Hühner in der Brutvorrichtung umfasst, und das Verabreichen dieses Futters an die geschlüpften Hühner in der Brutstätte das Verabreichen dieses Futters in der Brutvorrichtung während des Schlüpfens umfasst.

5. Verfahren nach Anspruch 4, umfassend die Aufzucht der neu-geschlüpften Hühner in der Brutvorrichtung während eines anfänglichen Aufzuchtzeitraums von zwischen 0 bis 4 Tagen.

6. Verfahren nach einem vorhergehenden Anspruch, worin das Verarbeiten der nicht-bebrütbaren Eier zu Futter in der Brutstätte das Sterilisieren der Eier und/oder Eikomponenten umfasst.

7. Verfahren nach Anspruch 6, worin das Verarbeiten des Chargenanteils der nicht-bebrütbaren Eier zu Futter in der Brutstätte einen oder mehrere der folgenden Schritte umfasst:
- Auswählen der Eier,
- Brechen der Eier, um eine Mischung aus Eierschale und Eikomponenten zu erhalten,
- Trennen der Eikomponenten in Schale, Eigelb und Eiklar,
- Desinfektion von Ei-Komponenten,
- Kochen der Eier,
- Verarbeiten der Eier in eine bröckelige Substanz,
- Pelletieren der Eier.

8. Verfahren nach Anspruch 7, umfassend Hinzufügen von Nährstoffen und/oder von anderen Tierfuttersubstanzen zur Ergänzung dieses Futters.

9. Verfahren nach einem vorhergehenden Anspruch, worin das Verabreichen dieses Futters an die geschlüpften Hühner in der Brutstätte das Verabreichen dieses Futters an die Hühner, die aus dem bebrütbaren Chargenanteil der Eier geschlüpft sind, umfasst.

10. Verfahren nach einem der vorhergehenden Ansprüche, worin das Verabreichen dieses Futters an die geschlüpften Hühner in der Brutstätte das Verabreichen dieses Futters an die geschlüpften Hühner der nachfolgenden oder der vorhergehenden bebrütbaren Charge der Eier umfasst.

11. Verfahren nach einem vorhergehenden Anspruch, worin das Ermitteln des Vorhandenseins eines bebrütbaren Hühnerembryos einen oder mehrere der folgenden Schritte: Herzschlagerkennung in Bezug auf ein Ei, Durchleuchten eines Eis, Wärmebildgebung eines Eis, Farbmessung eines Eis, Wiegen eines Eis, umfasst.

12. Verfahren nach Anspruch 11, umfassend, basierend auf dem Vorhandensein eines nicht-bebrütbaren Hühnerembryos und der Farbe oder des Gewichts eines Eis, das Unterteilen des nicht-bebrütbaren Chargenanteils in mindestens einen ersten nicht-bebrütbaren Chargenanteil und einen zweiten nicht-bebrütbaren Chargenanteil, worin die Eier des ersten nicht-bebrütbaren Chargenanteils nicht verfault sind und keinen Embryo oder einen Embryo von kleiner als etwa 20 mm enthalten, und Eier des zweiten nicht-bebrütbaren Chargenanteils verfault sind und/oder einen Embryo von größer als etwa 20 mm, enthalten.

13. Verfahren nach Anspruch 12, umfassend das Verarbeiten des ersten nicht-bebrütbaren Chargenanteils zu Futter.

## Revendications

1. Méthode (I) pour produire des poulets dans une écloserie, la méthode comprenant :
- l'incubation d'un lot d'oeufs (3) dans un dispositif d'incubation pendant une période d'incubation comprise entre environ 7 et environ 20 jours, et ensuite
- pour chaque oeuf du lot, la détection (4) de la présence d'un embryon de poulet pouvant éclore,
- sur la base de la présence d'un embryon de poulet pouvant éclore, la division du lot d'oeufs en au moins une partie de lot pouvant éclore et une partie de lot ne pouvant pas éclore,
- l'éclosion (7) de la partie de lot pouvant éclore dans un dispositif d'éclosion jusqu'à l'éclosion ;
- l'élevage de poulets éclos provenant de ladite partie de lot pouvant éclore,
**caractérisée en ce que** la méthode comprend, au niveau de l'écloserie, le traitement de la partie de lot ne pouvant pas éclore en alimentation et l'administration (9a) de ladite alimentation dans l'écloserie aux poulets éclos pendant l'élevage.

2. Méthode selon la revendication 1, comprenant l'administration uniquement de ladite alimentation dans l'écloserie, à des poulets éclos pendant l'élevage, de telle sorte que des nutriments supplémentaires ne sont pas requis au moins pendant une période initiale d'élevage d'environ 1 à 4 jours.

3. Méthode selon une revendication précédente, dans laquelle la méthode consiste dans l'écloserie à traiter la partie entière de lot ne pouvant pas éclore et à administrer ladite alimentation dans l'écloserie aux poulets éclos pendant l'élevage de sorte qu'aucun flux d'oeufs ou d'alimentation ne quitte l'écloserie.

4. Méthode selon une revendication précédente, dans laquelle l'élevage de poulets éclos provenant de ladite partie de lot pouvant éclore comprend l'élevage des poulets nouvellement éclos dans le dispositif d'éclosion, et l'administration de ladite alimentation dans l'écloserie aux poulets éclos comprend l'administration de ladite alimentation dans le dispositif d'éclosion pendant l'éclosion.

5. Méthode selon la revendication 4, comprenant l'élevage de poulets nouvellement éclos dans le dispositif d'éclosion pendant une période initiale d'élevage comprise entre 0 et 4 jours.

6. Méthode selon une revendication précédente, dans laquelle dans l'écloserie, le traitement de la partie de lot ne pouvant pas éclore en alimentation comprend la stérilisation d'oeufs et/ou de composants d'oeuf.

7. Méthode selon la revendication 6, dans laquelle dans l'écloserie, le traitement de la partie de lot ne pouvant pas éclore en alimentation comprend une ou plusieurs des étapes suivantes :
- sélectionner des oeufs,
- casser des oeufs pour obtenir un mélange de coquilles d'oeuf et de contenus d'oeuf,
- séparer les contenus d'oeuf en coquille, jaune et albumine,
- désinfecter des composants d'oeuf,
- cuisiner des oeufs,
- traiter des oeufs en une substance friable,
- comprimer des oeufs.

8. Méthode selon la revendication 7, comprenant l'ajout de nutriments et/ou autres substances d'alimentation animale pour compléter ladite alimentation.

9. Méthode selon une revendication précédente, dans laquelle l'administration de ladite alimentation dans l'écloserie à des poulets éclos comprend l'administration de ladite alimentation à des poulets éclos provenant de ladite partie de lot d'oeufs pouvant éclore.

10. Méthode selon une revendication précédente, dans laquelle l'administration de ladite alimentation dans l'écloserie à des poulets éclos comprend l'administration de ladite alimentation à des poulets éclos provenant d'un lot d'oeufs suivant ou précédent.

11. Méthode selon une revendication précédente, dans laquelle la détection de la présence d'un embryon de poulet pouvant éclore comprend un ou plusieurs procédé parmi une détection de battements de coeur relative à un oeuf, un mirage d'un oeuf, une imagerie thermique d'un oeuf, une mesure de couleur d'un oeuf, une pesée d'un oeuf.

12. Méthode selon la revendication 11, comprenant, en se basant sur la présence d'un embryon de poulet ne pouvant pas éclore et sur une couleur ou un poids d'un oeuf, la division de la partie de lot ne pouvant pas éclore en au moins une première partie de lot ne pouvant pas éclore et une seconde partie de lot ne pouvant pas éclore, dans laquelle des oeufs de la première partie de lot ne pouvant pas éclore ne sont pas pourris et ne contiennent pas d'embryon ou un embryon inférieur à environ 20 mm, et des oeufs de la seconde partie de lot ne pouvant pas éclore sont pourris et/ou contiennent un embryon plus grand qu'environ 20 mm.

13. Méthode selon la revendication 12, comprenant le traitement de la première partie de lot ne pouvant pas éclore en alimentation.
